# EUROPEAN PATENT SPECIFICATION

(11) **EP 0 720 615 B1**
(45) Date of publication and mention of the grant of the patent: **26.07.2000**
(21) Application number: 94927002.9
(22) Date of filing: 21.09.1994
(51) Int. Cl.: C07H 21/00, C12Q 1/68

(54) **ELONGATION, AMPLIFICATION AND SEQUENCE DETERMINATION USING A CHIMERIC OLIGONUCLEOTIDE**
ELONGATION, AMPLIFIKATION UND SEQUENZIERUNG UNTER VERWENDUNG EINES CHIMÄREN OLIGONUCLEOTIDS
REACTION D'ELONGATION, D'AMPLIFICATION ET DE DETERMINATION DE SEQUENCES UTILISANT UN OLIGONUCLEOTIDE CHIMERIQUE

(30) Priority: 21.09.1993 EP 93307455
(43) Date of publication of application: 10.07.1996
(73) Proprietor: Amersham Pharmacia Biotech UK Limited, Little Chalfont, Buckinghamshire HP7 9NA (GB)
(72) Inventor: REEVE, Michael, Alan, Henley-on-Thames Oxon RG9 1TE (GB); BROWN, Tom, Edinburg EH10 6UR (GB)
(74) Representative: Pennant, Pyers
(86) International application number: GB9402053
(87) International publication number: WO9508556

(56) References cited:
- EP-A- 0 224 126
- EP-A- 0 411 186
- EP-A- 0 546 761
- WO-A-92/20703
- DE-A- 4 110 085
- NUCLEIC ACIDS RESEARCH., vol.20, no.14, 25 July 1992, ARLINGTON, VIRGINIA US pages 3551 - 3554 A.SKERRA
- TRENDS IN BIOTECHNOLOGY, vol.11, September 1993, CAMBRIDGE GB pages 384 - 386 O.BUCHARDT ET AL.

## Description

There are a series of compounds which have standard nucleic acid bases attached to nonstandard polymer backbones (these backbones can be nucleic acid like; eg. phosphorothioate- or methylphosphonate-linked deoxyribose; or they can be very different; eg. polyamide). If the interbase spacing is correct, these compounds are capable of base pairing reactions analogous to normal nucleic acids. Interest in these compounds currently centres on their use in either therapeutics or diagnostic imaging.

The chemical and hybridization properties of some of these compounds are particularly interesting. A number of novel and inventive uses of these compounds as tools in Molecular Biology (as opposed to therapeutic and diagnostic pharmaceuticals as above) are presented herein. The synthesis and use of non-standard backboned nucleic acids has been described in W0 92/20702 and WO 92/20703. More recently W0 93/12129 and W0 93/13121 described the synthesis and some uses of similar molecules.

Described herein for use in the invention are chimeric nucleic acid/nucleic acid analogue molecules comprising a first portion, said first portion comprising a nonstandard backboned oligonucleotide having at least one amide linkage and a second portion, said second portion comprising an acceptor end which is a chemical functionality capable of acting as acceptor for the formation of a phosphodiester bond.

The preferred backbone for the nonstandard backboned oligonucleotide is polyamide. The oligonucleotide preferably comprises peptide nucleic acid (PNA).

Preferably, the nonstandard backboned oligonucleotide will contain at least two monomers, and more preferably three or more monomers. These may be monomers of PNA.

The chimeric molecules may be alternatively backboned nucleic acids that have a few normally backboned nucleotides at the 3' end. Such chimeric molecules will combine the altered properties associated with the nonstandard backbone with an ability to prime DNA synthesis from a normal template-bound 3' end. Some of these alternatively backboned nucleic acids are capable of dsDNA strand invasion below the target dsDNA melting temperature, somewhat like ssDNA/RecA complexes (Science, 254, p1497, (1991)).

Therefore new methods of sequencing are possible. This is particularly the case for dsDNA. A suitable chimeric molecule is mixed with the dsDNA to be sequenced and then incubated with a DNA polymerase and nucleoside triphosphates and appropriate dideoxynucleoside triphosphate terminators. There should be no need to denature the dsDNA to be sequenced. This is a major advantage especially for automated sequencing systems. In principle the 3' end of the sequencing primer may have any chemical functionality capable of acting as acceptor for the formation of a phosphodiester bond. This may be an -OH group on the non-standard backboned molecule itself or a 3' -OH group if some normally backboned nucleotide(s) are required. Only one normally backboned nucleotide may be required but it is more likely that a length of 2 to 12 bases, more preferably 3 to 7 bases may be required. Work described in Biochemistry 29 1200-1207 (1990) shows that very short conventionally backboned primers may be utilised.

The invention therefore provides a method of performing a primer extension reaction by the use of
a) a target nucleic acid
b) a primer which is a chimeric nucleic acid/nucleic acid analogue molecule comprising a first portion, said first portion comprising a nonstandard backboned oligonucleotide and a second portion, said second portion comprising an acceptor end which is a chemical functionality capable of acting as acceptor for the formation of a phosphodiester bond, said chimeric molecule being capable of hybridising to part of the target
c) a supply of nucleotides
   which method comprises mixing reagents a), b) and c) in the presence of a chain extension enzyme under conditions to allow the chimeric molecule to hybridise to the target and extension of the chimeric molecule at the acceptor end to occur, giving an extension product.

The invention also provides a method of performing a chain termination reaction by the use of
a) a target nucleic acid
b) a chimeric nucleic acid/nucleic acid analogue molecule comprising a first portion, said first portion comprising a nonstandard backboned oligonucleotide and a second portion, said second portion comprising an acceptor end which is a chemical functionality capable of acting as acceptor for the formation of a phosphodiester bond, said chimeric molecule being capable of hybridising to part of the target
c) a supply of nucleotides
d) a chain termination agent
   which method comprises mixing reagents a), b) c) and d) in the presence of a chain extension enzyme under conditions to allow the chimeric molecule to hybridise to the target and extension of the chimeric molecule at the acceptor end to occur, so as to produce terminated extension products, which terminated extension products are separated to allow part of the nucleotide sequence of the target nucleic acid to be determined.

A further aspect of the invention is a method of determining the nucleotide sequence of a target nucleic acid, which method comprises performing a chain termination method as described above, using a chain termination agent for each of the four different nucleotides such that the nucleotide sequence of the target may be determined.

The preferred chimeric molecules for use as primers in the methods described herein are chimeric molecules discussed above. Thus, the primers preferably have at least one amide linkage in the backbone of the nonstandard backboned oligonucleotide.

Generally, a 6-mer is accepted as the smallest effective priming unit, although this can be reduced to 3 or 4 if very low temperatures are used. The chimeric molecule primers described herein are preferably 6 or more base units in length.

One or more of the reagents used in the various methods according to the invention may be labelled in ways which are known in the art.

There may be advantage in using an oligomer primer in which the majority (most or all) of the bases are pyrimidines. Such primers may prove to have enhanced strand invasion properties.

Whilst it will be appreciated that the use of chimeric molecules as herein described in Sanger chain-terminating sequencing is desirable, they may also be used in other related methods such as cycle sequencing.

The possibilities for using the chimeric molecules described herein and the advantages these molecules provide in use also apply to other techniques including amplification techniques eg. PCR. It should therefore be possible to perform isothermal amplification of nucleic acids using the molecules herein described. Quantification of products produced by PCR reactions may also be improved by these molecules. In existing methods the dsDNA product of the PCR reaction is denatured and mixed with an immobilised capture probe. Some reannealing of the PCR product is inevitable and these molecules will not be captured by the immobilised probe leading to a loss of sensitivity. Use of PNA molecules, for example, in the capture step will eliminate the need to denature the PCR product leading to greater capture and increased sensitivity (PNA oligomers are peptide nucleic acid molecules comprising nucleic acid bases attached to a peptide backbone through a suitable linker and are described in detail in WO 92/20702 and WO 92/20703). The binding may also be stronger. The immobilisation of the capture probe may also be easier to achieve using conventional methods used for proteins and peptides.

Annealing of chimeric molecules at higher temperatures to provide increased specificity and greater sensitivity in primer extensions, PCR and sequencing reactions is expected.

A normally backboned nucleotide or oligonucleotide could also be attached by its 3' end to the other end of the non standard backboned nucleic acid so as to provide a normally backboned 5' end and facilitate kinase labelling etc.

Non standard backboned molecules could also be used in sequencing methods based on primer walking and subsequent developments by Studier (Science 11 December 1992 p 1787) . This original method makes use of sequence information near the terminus of a previously sequenced fragment of DNA to generate a new primer that will allow the next contiguous piece of DNA to be sequenced. This process can be repeated a number of times. One disadvantage of this approach, especially in genome sequencing projects, is the time required to determine and synthesise the next appropriate primer. This is slow and expensive.

The method developed by Studier uses a presynthesised pool of all possible different hexamers (approximately 4,000) of known sequence. These are combined to form 12mers or 18 mers to make the required primer. The hexamers can be ligated together on their complementary template or held together using a single stranded binding protein or just allowed to hybridise adjacent to each other.

If smaller primer units could be used then the number requiring synthesis would be much smaller. For example if 5mers could be used then approximately 1,000 would be required resulting in significant cost savings. Unfortunately, small oligonucleotides do not hybridise strongly. The strong hybridisation properties of, for example, PNA oligomers or other non-standard backboned molecules overcomes this problem. A combination of random PNA molecules and random standard oligonucleotides can be used to derive more efficient primer-walking strategies. The use of PNA-DNA chimeras which would hybridise more efficiently and also be primers for DNA polymerases is also possible.

The following list indicates some of the main uses for the chimeric molecules described herein. It will be clear to those skilled in the art that many other uses exist.
1. Primers for polymerases (using chimeric molecules with normally backboned nucleic acids giving the 3' end: priming could also occur from strand invasion complexes formed by reaction with dsDNA targets, a strand displacing polymerase would be used for the extension) . This can also be extended to random primer labelling of dsDNA molecules using chimeric primers and strand invasion.
2. In situ hybridization with no need to denature the sample.
3. Primers/Probes that do not require dsDNA denaturation.
4. Improved sequencing by hybridization with short oligos.
5. Double strand sequencing without denaturation.
6. Isothermal PCR and related PCR alternatives.
7. Random primer labelling without denaturation.
8. Better probes for primer extension mapping procedures.
9. New labels and probes for multiplex sequencing.
10. Novel adapters/linkers for cDNA cloning.

### TERMINOLOGY:

By nucleic acid(s), we mean either DNA or RNA of any chain length which can be either wholly or partially single or double stranded unless otherwise specified.

By "normally backboned", we refer to phosphodiester linked deoxyribose (for DNA) or phosphodiester linked ribose (for RNA) as the backbone to which the base residues (A, C, G and T for DNA and A, C, G and U for RNA) are linked.

By "nonstandard backboned", we refer to any polymers other than the normal phosphodiester linked deoxyribose (for DNA) or phosphodiester linked ribose (for RNA) as the backbone to which the base residues (A, C, G and T for DNA and A, C, G and U for RNA) are linked. Unless otherwise stated, the only requirement for the nonstandard backboned polymer is that the interbase spacings are suitable for the formation of appropriate hydrogen bonds (Watson and Crick or triple helical or Hoogsteen type) with a normally backboned nucleic acid target. Examples of nonstandard backbones are phosphorothioate linked deoxyribose, phosphorothioate linked ribose, methylphosphonate linked deoxyribose, methylyphosphonate linked ribose and polyamide. It will be immediately obvious to one skilled in the art that there are many other possible backbone polymers allowing the correct interbase spacing and that this allows for a number of different chemical and physical properties specific to the backbone moiety to be exploited whilst preserving the ability to bind to a complementary nucleic acid base sequence by hydrogen bonding.

It is also to be understood herein that chimeric molecules comprising nonstandard backboned nucleic acids with normally backboned nucleic acid ends, for ligation, priming, labelling and other such applications known to those skilled in the art, are also possible. Such chimeric molecules are to be included in the Claims wherever the term "nonstandard backboned" is used unless otherwise specified. In chimeric molecules the normally backboned sequence may be directly linked to the non-standard backboned sequence. It is also possible to include a small linker group between the two sequences.

By hybridization, we mean the sequence specific binding between a probe (with A, C, G and T residues or A, C, G and U residues attached to a normally backboned or nonstandard backboned polymer as specified) and a target nucleic acid. For a wholly or partially double stranded target, the sequence specific binding may also occur in a double stranded region by a process referred to herein as "strand invasion". Strand invasion is where the sequence specific binding of probe occurs under conditions in which the target strands do not normally separate from each other (for example at temperatures below the melting temperature of the target in a given solvent at a given ionic strength) . Strand invasion does not normally occur with normally backboned probes. The application of this strand invading property of some nonstandard backboned nucleic acid probes to improve existing and create novel Molecular Biology applications is a major inventive step.
Figures 1 to 5 show HPLC traces for the DNA and PNA/DNA molecules synthesised in Example 6:
Figures 1a and 1b - 15-mers of DNA and PNA/DNA, respectively;
Figures 2a and 2b - 12-mers of DNA and PNA/DNA, respectively;
Figures 3a and 3b - 9-mers of DNA and PNA/DNA, respectively;
Figures 4a and 4b - 7-mers of DNA and PNA/DNA, respectively;
Figures 5a and 5b - 5-mers of DNA and PNA/DNA, respectively.

### EXAMPLES

### Example 1

### Synthesis of chimeric molecule with single normal backboned base

0.5mg of PNA₁₅₄-NH₂ (H-CAT CTA GTG A-LysNH₂), synthesised according to the methods disclosed in WO 92/20702 and WO 92/20703, was mixed with 0.1mg 5'-amino thymidine in 0.02ml 33%DMSO, 100 mM Tris-HCl pH 7.4. The reaction was started by the addition of 0.05mg subaric acid bis(N-hydoxysuccinimide) ester and incubated at room temperature for 24 hours.

### Synthesis of chimeric molecule with longer standard backboned sequence

0.5mg ^{5'} H₂NTCG CAC TGC ATC ^{3'} (standard backbone) in 0.25ml 100mM Tris-HCl pH 7.4 was mixed with 0.5mg subaric acid bis(N-hydroxysuccinimide) ester in DMSO for 5 minutes. The reactants were purified by gel filtration, freeze dried and resuspended in 0.1ml 100mM Tris-HCl pH 7.4. 0.5mg PNA₁₅₄ in 0.025ml Tris-HCl pH 7.4 was added and incubated for 24 hours at room temperature.

### Example 3

### PNA-DNA PRIMER EXTENSION ASSAY - GENERALISED PROTOCOL

### Annealing conditions

The PNA-DNA primer synthesised as in examples 1 and 2 is diluted, 0 , 1/10 1/100 and 1/1000 in 50mM tris,pH 7.5 containing 50mM NaCl and 7mM MgCl₂.

The control DNA 11mer primer is diluted to 10pmole/µl in the same buffer.

The PNA-DNA primer and the control primer are mixed with equal volumes of 2pmole/µl template oligo to give 1pmole/µl template concentration.

Annealing is performed by boiling the mixtures for 3 minutes and then leaving the solutions to cool to room temperature over a period of approximately 1 hour .

### Primer Extension Assay

The extension reactions are carried out containing the PNA-DNA or the DNA primed template with 200µM dATP,dGTP, dTTP and 20µCi of alpha ³²P dCTP with exonuclease free Klenow polymerase.

The reactions are incubated at 37°C for 20 minutes then dCTP is added to a final concentration of 200µM. The reactions are incubated for a further 10 minutes then terminated by the addition of EDTA. Free nucleotide is removed by spin column centrifugation. The results can be analysed by conventional denaturing polyacrylamide electrophoresis.

### Example 4

The synthesis of the thymine PNA monomer is outlined in scheme 1.

All solvents were of HPLC grade; DCM and pyridine was distilled over CaH₂; MeOH was distilled over Mg and I₂; EtOH was distilled over CaO then Mg and I₂; DMF was of peptide synthesis grade and was not further purified; anhydrous acetonitrile was purchased from Applied Biosystems Inc. (ABI). All other chemicals were supplied by Aldrich. ¹H and ¹³C n.m.r. were recorded on a Brucker 250AC and Brucker 200WP spectrometer. Positive ion Fast Atom Bombardment (FAB) mass spectra were recorded on a Kratos MS50TC spectrometer using a thioglycerol matrix. Oligonucleotide synthesis was carried out on an ABI 394 DNA synthesiser. PNA/DNA synthesis (detritylation and cleavage from the solid support) was carried out on an ABI 380B DNA synthesiser.

Flash chromatography was carried out using silica gel 60 (Fluka). Thin layer chromatography (tlc) was carried out on aluminium sheets, silica 60 F₂₅₄. 0.2mm layer (Merck) using the following solvent systems; A=nBuOH/AcOH/H₂O (3:1:1 v:v), B=DCM/EtOAc (1:1 v/v), C=DCM/MeOH (9/1 v/v). Products were visualised using ninhydrin (1% w/v in EtOH), with heating for 5 mm. ,short wave UV lamp(254nm) or via iodine oxidation.

### 1-Thyminylacetic acid (I)

Thymine (10.0g, 79.3 mmoles) was dissolved in water (50ml) containing KOH (17.1g. 0.30 moles) at 40°C. A solution of BrCH₂CO₂H (16.5g, 1.5 eq.) in water (25ml) was added dropwise over 30 min, and the reaction mixture heated at 40°C for 2h. The solution was cooled to room temperature, the pH adjusted to 5.5 (c. HCl) then stored at -4°C for 2h. The precipitate formed was removed by filtration and the filtrate adjusted to pH 2 (c. HCl). The precipitate formed was isolated by filtration and dried over P₂O₅ (12.5g. 86%). R_{f}=0.1(A), FAB MS 185 (M+1)⁺, ¹H n.m.r. (200MHz, DMSO); 1.74(s,3H,Me), 4.36(s,2H,CH₂), 7.48(s1H,Ar-H), 11,4(s,1H,NH). ¹³C n.m.r. (50MHz, DMSO); 12.1(Me), 48.7(CH₂), 108.6(C5), 142.1(C6), 151.2(C2), 164.7(C4), 169.9(CO₂H).

### 3-t-butoxycarbonylamino-1,2-propanediol (II)

3-amino-1,2-propanediol (10g, 0.11moles) was dissolved in water (250mL). The solution was cooled to 0°C and Boc anhydride (25g. 0.12 moles) added in one portion. The reaction mixture was brought to room temperature and the pH maintained at 10.5 using NaOH (2M) until the pH remained constant. The reaction mixture was stirred for a further 16h. at room temperature then EtOAc (200mL) added and the pH adjusted to 2.5 using HCl (4M) at 0°C. The phases were separated and the aqueous phase extracted with EtOAc (10x150mL). The combined organic phases were dried (MgSO₄) then evaporated *in vacuo* to yield V as an oil. The product solidified upon freezing (20.4g, 97%). R_{f}=0.75(A), 0.60(B), FAB MS 192 (M+1)⁺, ¹H n.m.r. (200MHz, CDCl₃); 1.39(s,9H,Boc Me), 3.14(broad, 2H, CH₂), 3.52(m,2H,CH₂), 3.68(m,1H,CH), 4.75(s,2H,2xOH), 5.52(m,1H,NH). ¹³C n.m.r. (50MHz, CDCl₃); 28.17(Me), 42.55(NCH₂), 63.48(CH₂), 71.08(CH), 79.76(quaternary C), 157.1(C=O).

### t-butoxycarbonylaminoacetaldehyde (III)

The diol V (5g, 26.1mmoles) was dissolved in water (50mL) and NaIO₄ (6.8g, 1.2eq.) added. The reaction mixture was stirred at room temperature for 3h., filtered, then the aqueous solution extracted with DCM (5x100mL). The combined organic phases were dried (MgSO₄) then evaporated to dryness *in vacuo* to yield the aldehyde as a colourless oil (3.9g, 94%). The product was used without further purification in the next step. R_{f}=0.7(B), 0.15(A); ¹H n.m.r. (200MHz, CDCl₃); 1.38(s,9H,Boc), 3.98(s,2H,CH₂), 5.33(b,1H,NH), 9.56(s,1H,CHO). ¹³C n.m.r (50MHz, CDCl₃); 28.0 (Me), 51.1(CH₂), 79.9(qC), 155.6(Boc C=O), 197.4(CHO).

### N-(t-butoxycarbonylaminoethyl)-glycine ethyl ester (IV)

The aldehyde **III** (5.13g, 32.2 mmoles) was dissolved in MeOH (100ml) and added to a mixture of ethyl glycinate (HCl salt, 11.2g. 2.5eq.) and Na BH₃CN (2.02g, 1eq.). The reaction mixture was stirred at ambient temperature for 16h. then the solvent removed *in vacuo*. The residue was dissolved in water (100ml) and the pH adjusted to 8.0 using NaOH (2M). The aqueous phase was extracted with DCM (5x150ml), the combined organic fractions dried (MgSO₄), then evaporated *in vacuo* to an oil which was purified by Kugelrohr distillation (6.92g. 87%) R_{f}=0.55(B), 0.15(C), ¹Hn.m.r. (250MHz,CDCl₃); 1.09(t,3H,CH₃), 1.25(s,9H,BocMe),2.53(t,2H,CH₂), 2.90(b,1H,NH), 3.46(t,2H,CH₂NH), 3.54(s,2H,CH₂),4.00(q,2H,CH₂CH₃),5.46(b,1H,carbamateNH). ¹³Cn.m.r.(50MHz,CDCl₃); 13.69(CH₃CH₂), 27.91(BocMe), 39.6, 42.6, 48.27, 60.36(all CH₂), 78.58(quat. C), 156.1(BocC=O), 172.4(C=O).

### N-(t-butoxycarbonylaminoethyl)-N-(thyminylacetyl)-glycine-ethyl ester (V)

Bocaminoethylglycine ethyl ester (IV, 2.75g, 11mmoles) was dissolved in DMF (12mL) and thyminylacetic acid (I, 2.05g, 1eq.) was added. On dissolution of the acid DCM (10mL) was added, the reaction mixture cooled to 0°C and DCC (2.49g. 1.2eq.) added. The reaction was stirred at 0°C for 1h. then for a further 2h. at room temperature. The precipitated DCU was removed by filtration, washed with DCM (2x30mL) and a further volume of DCM (150mL) added. The combined organic phases were washed with NaHCO₃ (0.5M, 3x100mL), citric acid (10%w/v, 2x100mL), and NaCl solution (sat., 1x100mL), dried (MgSO₄) then evaporated to dryness *in vacuo* to yield a brown oil. The crude product was purified by silica gel chromatography eluting with DCM/MeOH(1-3%) to yield the product as a white foam (1.86g, 41%), R_{f}=0.55(A),0.15(C) FAB MS 413 (M+1)⁺, ¹H n.m.r. (250MHz,CDCl₃); (some of the signals are split into major and minor peaks due to restricted rotation around the secondary amide bond.) 1.18(t,3H,MeCH₂), 1.35(s,9H,BocMe), 1.81(s,3H,TMe), 3.25(m,2H,CH₂), 3.50(m,2H,CH₂), 4.00(s,2H,NCH₂CO₂Et), 4.39(s,mi,CH₂CON), 4.53(s,mj,CH₂CON), 5.76(b,1H,BocHN), 6.99(s,mj,Ar-H), 7.0(s,mi,Ar-H), 10.0(s,mj,NH), 10.05(s,mi,NH). ¹³C n.m.r. (50MHz,CDCl₃); 12.1(CH₃CH₂), 13.8(T-Me), 28.14(BocMe), 38.4, 47.6, 48.4, 48.7, 61.4(all CH₂), 79.6(quat.C), 140.84(C6), 151.0, 155.9, 164.4, 167.2, 169.4(all C=O).

### N-(Monomethoxytritylaminoethyl)-N-(thyminylacetyl)-glycine-ethyl ester (VI)

1-(Bocaminoethylglycine)-thymine ethyl ester (VII, 2.0g. 4.85mmoles) was dissolved in TFA (10mL). The reaction mixture was evaporated to dryness *in vacuo* then the residue coevaporated with toluene (5x10ml) then pyridine (2x10mL). Monomethoxytrityl chloride (1.65g. 1.1eq.), 4-pyrrolidinylpyridine (25mg) and pyridine (10mL) were added and the solvent was removed *in vacuo* once more. Pyridine (10mL) was added and the reaction mixture was stirred for 16h. Water (30mL) was added and the reaction mixture extracted with DCM (3x70mL). The combined organic phases were dried (MgSO₄), then evaporated to dryness *in vacuo* to yield an oil. The product was isolated by column chromatography using DCM/MeOH(0-3%) as the eluant (2.55g. 90%). The SiO₂ was pre-equilibrated with DCM containing NEt₃ (1%v/v) to prevent detricylation. R_{f}=0.2(A), 0.1(A); FAB MS 585 (M+1)⁺, ¹H n.m.r. (250MHz, CDCl₃); (some of the signals were split into major and minor peaks due to restricted rotation about the secondary amide bond) 1.18(t,3H,MeCH₂), 1.90(s,3H,T-Me), 2.38(m,2H,CH₂NCO), 3.52(m,2H,TrNH-CH₂). 3.76(s,3H,OMe), 3.90(s,mj,CH₂CON), 4.02(s,mi,CH₂CON), 4.10(q.2H,CH₂Me), 4.25(s,mj,CH₂CO₂Et), 4.31(s,mi,CH₂CO₂Et), 6.82(s,mi,H6), 6.85(s,mj,H6), 7.2-7.4(m,Ar-H). ¹³C n.m.r.(50MHz, CDCl₃); 12.2(CH₃CH₂), 3.9(T-Me), 41.7, 48.0, 48.5, 50.0, 61.3(all CH₂), 55.0(OMe), 110(CPh₃), 123-128(m,Ar-H), 137.4(quatC), 140.8(C6), 145.6(quatC), 149.5(quatC), 151.0(COMe), 157.8, 164.1, 167.3, 168.8(allC=O).

### N-(Monomethoxytritylaminoethyl)-N-(thyminylacetyl)-glycine-(VII)

The ester XI (1.34g, 2.3mmoles) was dissolved in MeOH (60mL) and NaOH (2M, 40mL) added. The reaction mixture was stirred at room temperature for 2h. then DOWEX (pyridinium form) was added until a pH of 7 was obtained. The suspension was filtered and the resin washed with MeOH (3x50mL). The filtrate was evaporated to dryness *in vacuo* to yield XI (1.4g. 96%). R_{f}=0.05(B),0(C); ¹H n.m.r. (200MHz,CDCl₃); (some of the signals were split into major and minor peaks due to restricted rotation about the secondary amide bond) 1.75(s,3H,T-Me), 2.21(m,2H,CH₂), 3.49(m,2H,CH₂), 3.76(s,3H,OMe), 3.85(s,mj,CH₂CON), 4.05(s,mi,CH₂CON), 4.52(s,mj,CH₂CO₂H), 4.85(s,mi,CH₂CO₂H), 6.85(s,mj,H6), 6.90(s,mi,H6), 7.2-7.5(m,Ar-H). ¹³C n.m.r.(50MHz,CDCl₃); 12.08(T-Me), 33.48, 42.15, 48.23, 48.38 (all CH₂), 55.07(OMe), 113.2(CPh₃), 126-131(m,Ar-H), 137.4, 146.0, 149.8 (all quat C), 150.8(COMe), 158.9, 164.4, 168.3, 173.3(C=O).

### Example 5

The nucleoside 5'-monomethoxytritylamino-5'-deoxy thymidine phosphoramidite was synthesised as shown in scheme 2 and was used as the initial linker in the PNA DNA chimeric molecule.

### 5'-azido-5'deoxy thymidine (VIII)

Thymidine (14.0g, 58.0mmoles) was stirred in DMF (175ml) and PPh₃ (18.2g,1.2eq.), NaN₃ (11.3g, 3eq.), and CBr₄ (23.0g. 1.2eq.) added at 0°C. The reaction mixture was stirred at 0°C for 1h. then ambient temperature for 16h. The reaction mixture was poured onto NaHCO₃ (0.5M, 350ml) and extracted with DCM (6x150ml). The combined organic phases were dried (MgSO₄) then evaporated to an oil which was purified by SiO₂ chromatography (0-4% MeOH/DCM , 7.73g, 50%). R_{f}=0.1(C), 0.8(A), FABMS (M+1) 268, ¹H n.m.r. (200MHz, D6-DMSO) 1.78(s,3H,T-CH₃), 2.10(m, 1H,2''H), 2.26(b,1H,2'H), 3.56(d,2H, 5''H), 3.85(m,1H,4'H), 4.20(m,1H,3'H), 5.42(d,1H,3'OH), 6.21(t,1H,1'H) 7.5(s,1H,6H), 11.34(s,1H,NH). ¹³C n.m.r.(50MHz, D6-DMSO); 12.2(CH₃), 33.9(CH₂), 51.8(CH₂), 70.9,72.1(CH), 136.2(C6), 150.6, 163.8(C=O).

### 5'-N-(4-Methoxytrityl)amino-5'-deoxythymidine (X)

To the azido compound (7.73g. 29mmoles) in pyridine (100mL) was added PPh₃ (15.2g, 1.2 eq.). The reaction mixture was stirred at room temperature for 16h. then water (20ml) added. After stirring for 1h. the reaction mixture was poured onto water (300ml) and stirred for a further 16h. The suspension was filtered and the aqueous phase washed with EtOAc (3x150ml). The 5'-amino-5'-deoxy-thymidine was isolated by freeze drying (4.1g. 58%).

A fraction of the 5'-amino-5'-deoxy-thymidine (1.34g. 5.55 mmole) was coevaporated with pyridine (2x20ml) then dissolved in pyridine (40ml). 4-pyrollidinopyridine (30mg) and p-anisoyldiphenylmethyl chloride (2.05g, 1.2eq.) were added, the reaction mixture stirred at room temperature for 16h. then poured onto NaHCO₃ solution (0.5M,30ml). The aqueous phase was extracted with DCM (3x50ml), the combined organic phases dried (MgSO₄) then evaporated to dryness *in vacuo*. The resultant oil was purified by column chromatography (SiO₂ preequilibrated with 1% NEt₃ in DCM eluting with 0-3% MeOH/DCM). (2.02g,71%) R_{f}=0.5(C), 0.2(B). FABMS (M+1)=514 ¹H n.m.r. (200MHz, D6-DMSO); 1.69(d,3H,CH₃), 2.01-2.34(m,4H,2'H,5'H), 2.64(t,1H,5'NH), 3.72(s,3H,OMe), 3.83(m,1H,4'H), 4.19(m,1H,3'H), 5.22(d,1H,3'-OH), 6.14(t,1H,1'H), 6.84 (d,2H,OMe), 6.87-7.43(m,1,3H,aromatic), 11.29(s,1H,NH). ¹³C n.m.r.(50MHz, D6-DMSO); 12.2(CH₃), 24.9(CH₂), 40.0(CH₂), 54.8(OMe), 71.6(CH), 84.0(CH), 85.9(CH), 112.8-129.4(aromatic CH), 134.9, 137.4, 145.6, 147.8, 157.5(all qC), 150.4(C=O), 164.1(C=O).

### 5'-N-(4-Methoxytrityl)amino-5'-deoxythymidine-3'-O-(2-cyanoethyl, N,N-diisopropylamino)phosphite (XI)

5'-N-(4-Methoxytrityl)amino-5'-deoxythymidine (0.44g, 0.85mmoles) and diisopropylammonium tetrazolide (30mg, 0.25eq.) were coevaporated with pyridine (2x5ml) then MeCN (5ml). The residue was dissolved in MeCN (5ml) then 2-cyanoethyl-N,N ,N',N'-tetraisopropylphosphorodiamidite (0.35ml, 1.4eq.) added and the reaction mixture stirred at room temperature for 16h. DCM (50ml) was added and the solution washed with NaHCO₃ (0.5M,20ml), dried (MgSO₄) then evaporated to an oil which was purified by SiO₂ chromatography (100% EtOAc, 0.30g, 49%). Rf=0.77 (100% EtOAc).

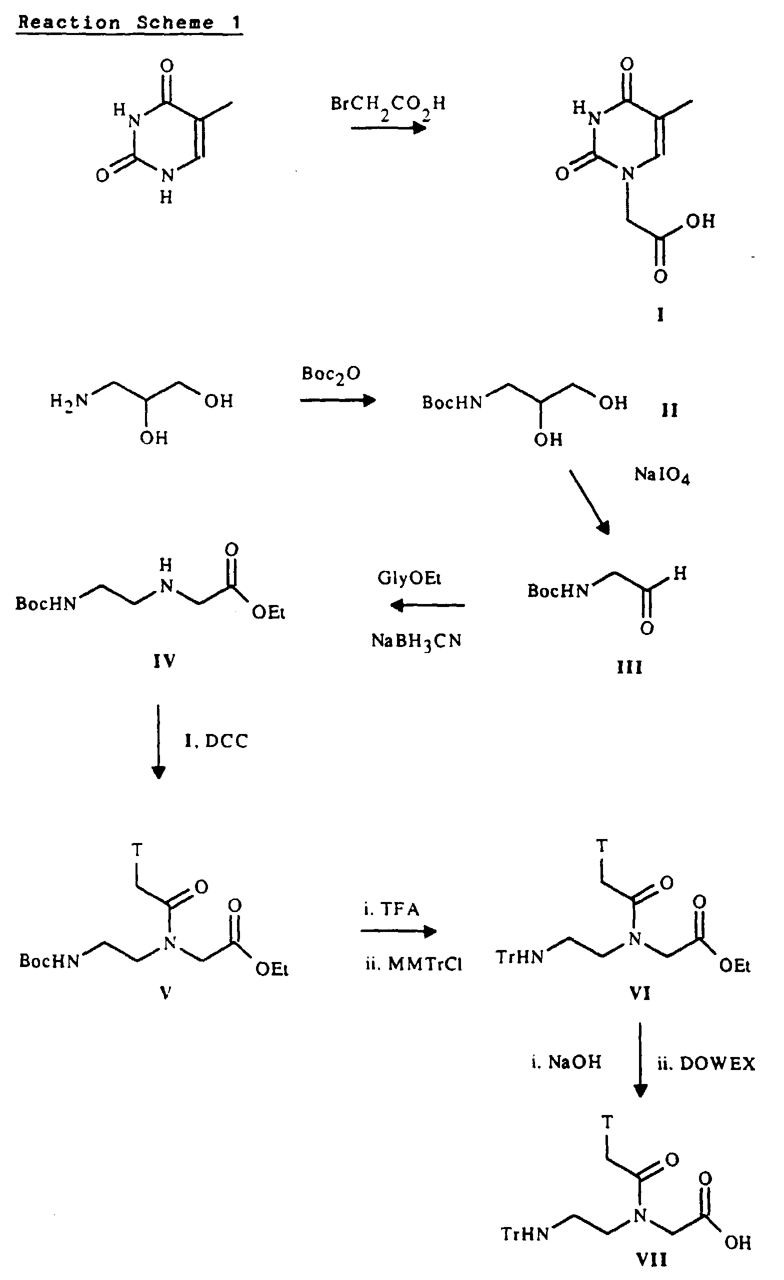

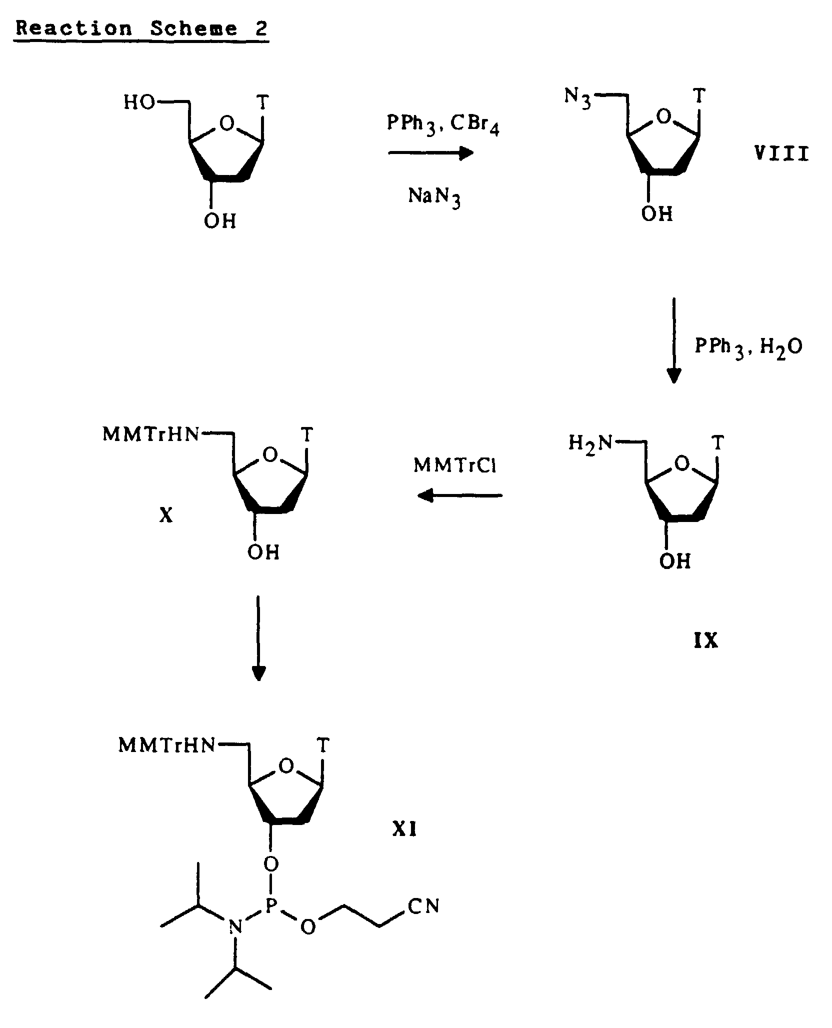

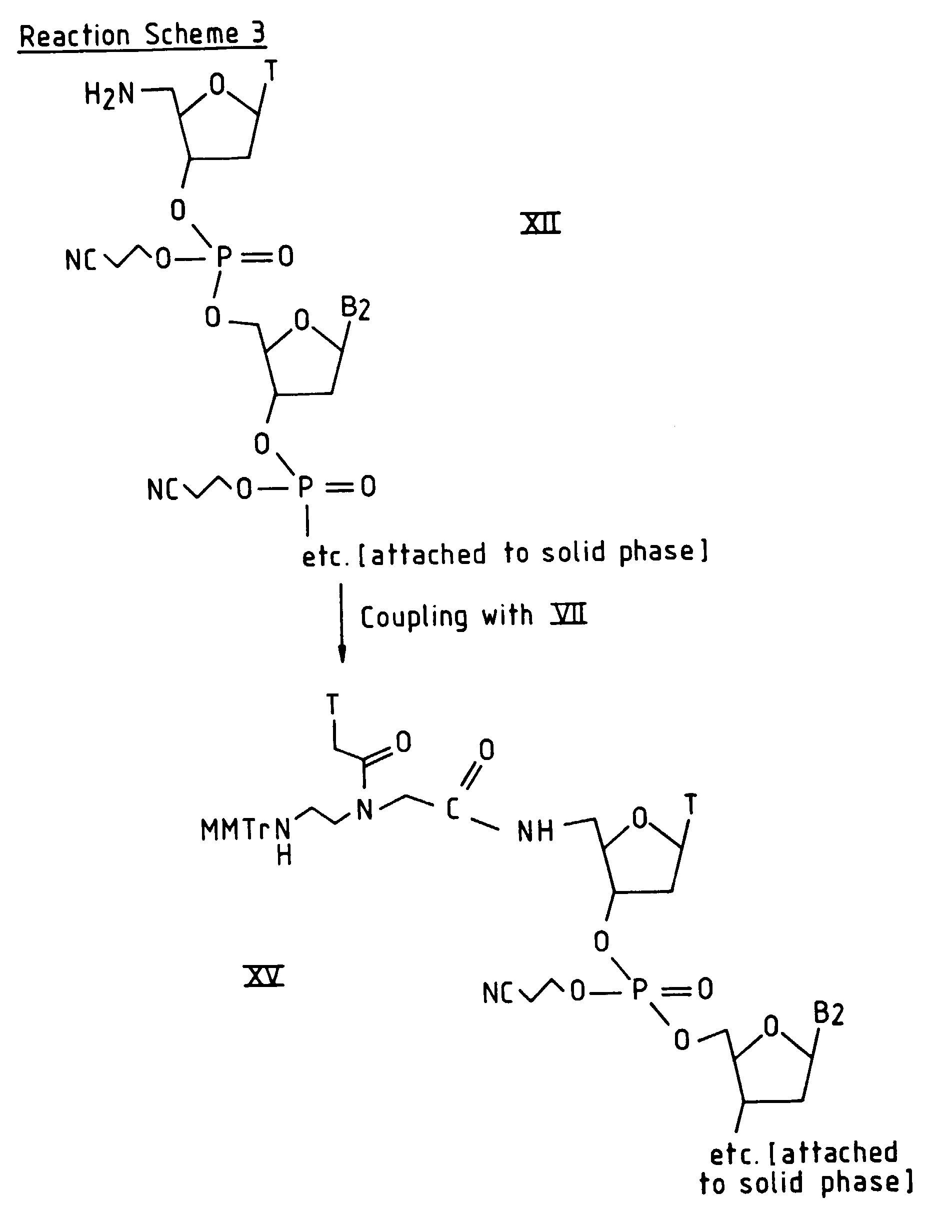

### Example 6

### Solid Phase Synthesis of PNA/DNA Chimeric Compounds

Chimeric molecules were made by initial synthesis of the DNA moiety using standard phosphoramidite chemistry and standard automated synthesis on an ABI 394 synthesiser. The final DNA monomer addition was a modified nucleoside phosphoramidite; a 5'-amino-5'-2'-dideoxy nucleoside derivative, protected at the 5'-end with a monomethoxytrityl group (XI in Reaction Scheme 2). This was deprotected at the 5'-end with trichloroacetic acid in the standard way and the solid phase bound oligonucleotide was then ready for the addition of the PNA monomers. The PNA section of the molecule was then added in a stepwise fashion using a manual solid phase methodology.

### Standard procedure for manual solid phase coupling

### Reaction Scheme 3 shows coupling of support bound DNA with PNA.

The trityl protecting group of the support bound DNA was cleaved using the appropriate cycle on an ABI 380B DNA synthesiser giving solid phase DNA with a 5'-amino group ready for a PNA coupling reaction XII. The PNA monomer VII (which is protected with a monomethoxy trityl group at the amino terminus) 280 mg was dissolved in DMF/Pyridine (1:1 v/v 4.4ml) to yield a 0.1M solution. An aliquot (0.1ml) was added to the peptide coupling reagent HBTU (18mg, 5eq.), and DECHA (20µl, 10 eq.) then manually passed repeatedly through the column containing the supported DNA for 1h. The coupling procedure was then repeated, to improve yield. The PNA monomethoxytrityl group was then removed on the solid phase using trichloroacetic acid and the next PNA coupling reaction was carried out as previously.

When the desired number of monomers had been added, the molecule was cleaved from the solid support using a standard cycle on the DNA synthesiser and the protecting groups removed by heating in NH₃ at 55°C for 5h. The product was purified by reverse phase HPLC. HPLC purification was carried out on a Gilson 303 system using a reverse phase octadecyl stationery phase. The PNA/DNA molecules were purified "trityl off " and the buffer system employed was; A=NH₄OAc (0.1M), B= MeCN (30% v/v)/NH₄OAc (0.1M). The flow rate was 3ml/min, detector 280nm.

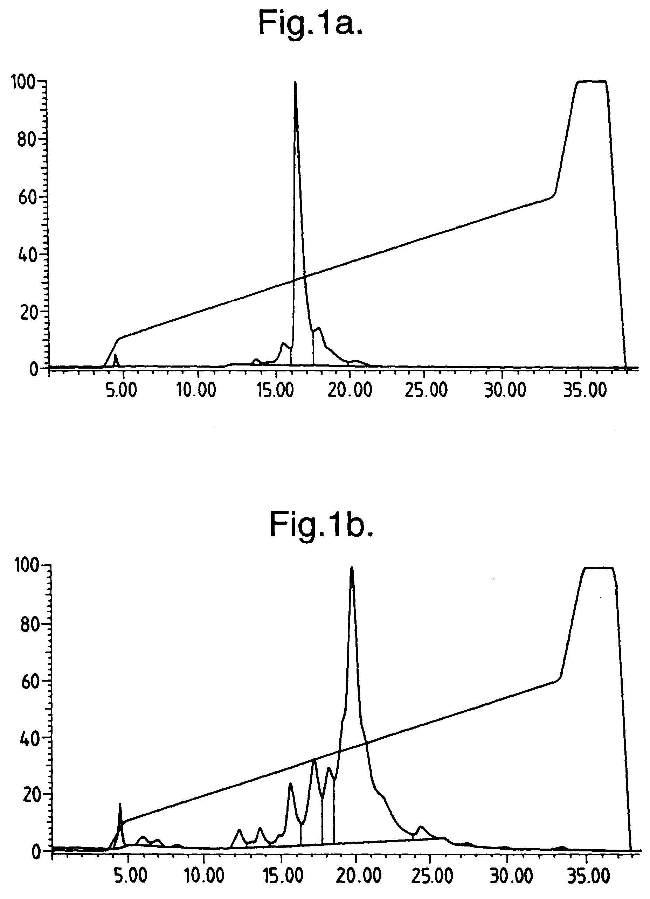

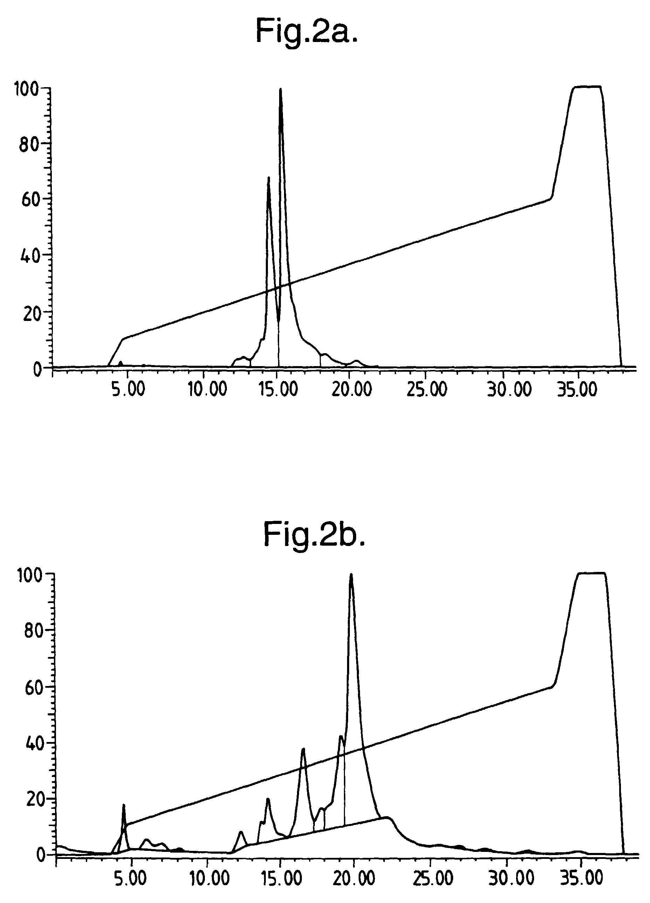

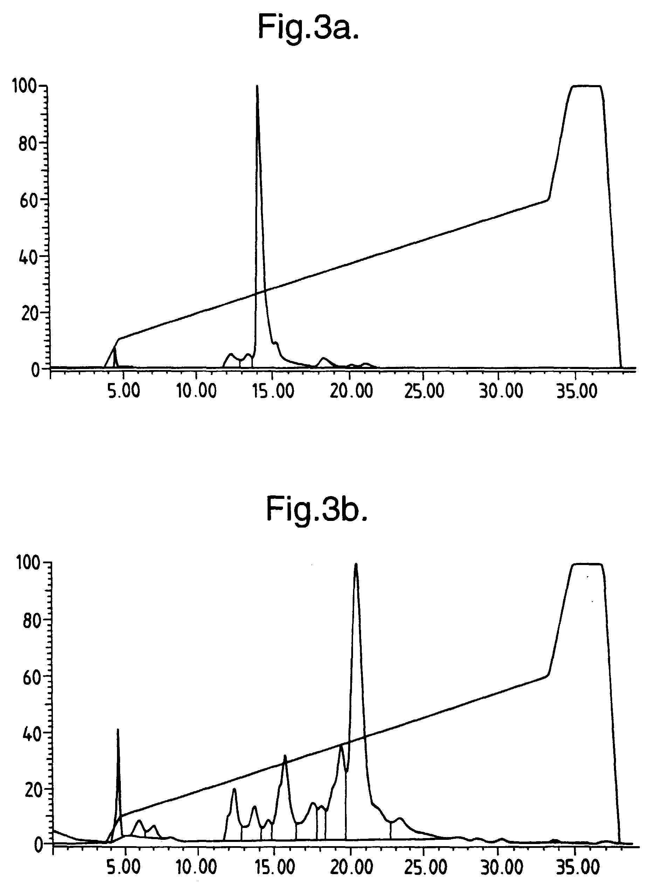

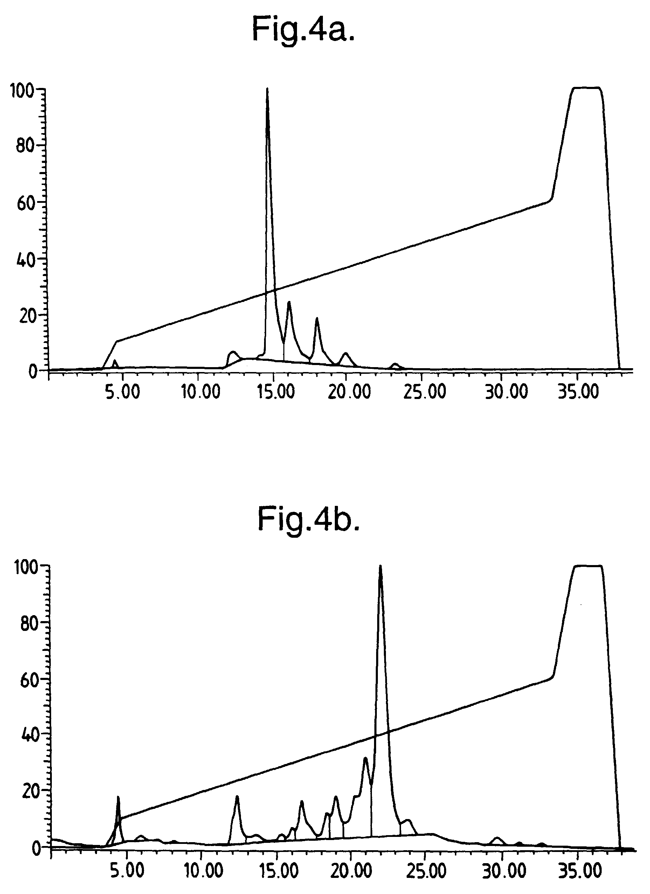

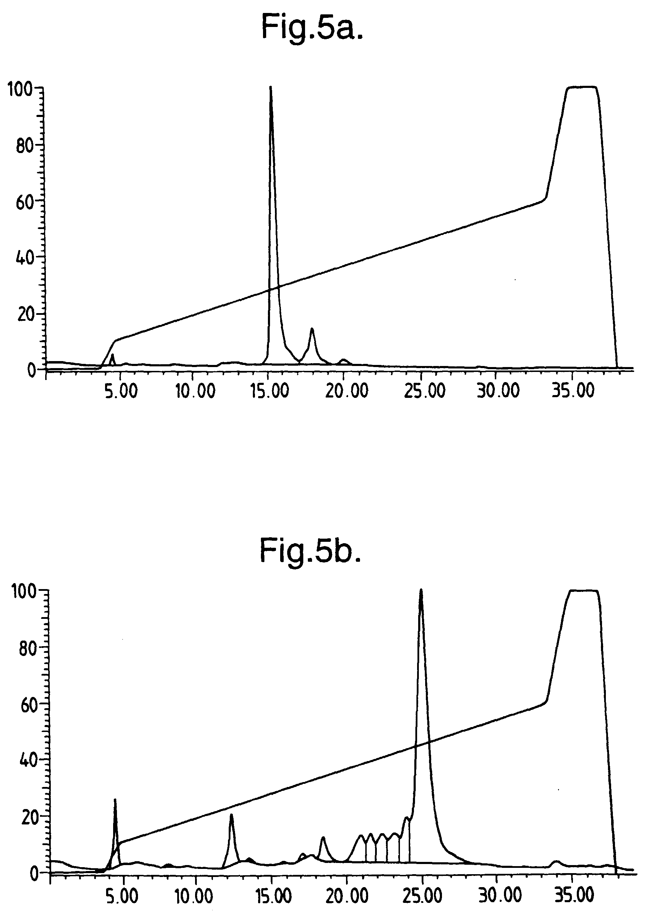

The PNA/DNA chimeric molecules in Table 1 below were synthesised according to the above method. PNA moieties appear in lower case and DNA bases in upper case. Equivalent DNA sequences with standard T nucleotides were also synthesised. Figures 1 to 5 show compared HPLC data for the PNA/DNA and DNA sequences. Clear peaks were obtained and in all cases mobility of PNA/DNA molecules was retarded compared to DNA molecules of equivalent length and sequence.

**Table 1**

| SIZE | SEQUENCE | FIGURE NO. |
|---|---|---|
| 15-mer | tttTAGAGTGTTGTT | 1b |
| 12-mer | tttTAGAGTGTT | 2b |
| 9-mer | tttTAGAGT | 3b |
| 7-mer | tttTAGA | 4b |
| 5-mer | tttTA | 5b |

### Example 7

### PNA-DNA Primer Extension Assay

### Annealing Conditions

The 18-mer DNA template comprised the following sequence:
5'-CTGAACAACACTCTAAAA-3'

The PNA-DNA primers were synthesised as described in example 6 and shown in Table 1 above.

The control primers comprised DNA only, having base sequences identical to the PNA-DNA primers described.

Annealing reactions were carried out in a total volume of 10µl in the presence of half strength Klenow buffer (1X Klenow buffer contains: 50mM Tris-Hcl (pH7.5), 5mM MgCl₂, 5mM β-Mercaptoethanol). The concentrations of primer and template were 5 and 1 pmol respectively.

Annealing was performed by boiling the reaction mixtures for 3 minutes and then leaving the samples at room temperature for 30 minutes.

### Primer Extension Assay

The extension reactions contained the contents of the annealing reactions described above (10µl each) together with 20µM dGTP, 20µM dATP, 20µM dTTP, 2.5µCi α-³² PdCTP (3000Ci/mmol, Amersham), 1.5X Klenow buffer, Klenow or exonuclease free Klenow (United States Biochemical) at 1 unit per reaction in a total volume of 20µl. (Klenow is a fragment of the enzyme DNA polymerase 1).

5mM d ATP α-S was included in reactions containing Klenow enzyme in order to prevent the 3'-5' exonuclease activity of the said enzyme. Reactions were incubated at 37°C for up to 30 minutes. Reaction products were analysed by polycrylamide gel electrophoresis or TLC as described below.

### Thin Layer Chromatography (TLC) Analysis System

2µl of each reaction mix was added to 3µl of stop solution (95% formamide, 20mM EDTA, 0.05% bromophenol blue and xylene cyanol FF). 1.0µl of each stopped reaction was then spotted onto a Schleicher and Schuell PEI-cellulose TLC plate 20mm up from the base of the plate. The plates were developed in a tank containing 1M KH₂PO₄. The plates were allowed to run until the solvent front had reached 10mm from the top of the plate. The plates dried and analysed on a Raytest TLC scanner. Labelled reaction products stayed on or close to the origin whereas the [α³²P] dNTP or excised [³²P] dNMP moved up the plate according to the base used and the number of phosphates.

### Denaturing Polyacrylamide gel electrophoresis (PAGE)

Page I™ Sequencing Gel Mix (19:1) from Boehringer Mannheim Corporation was diluted according as described by the manufacturer to give an 18% polyacrylamide gel mix containing 7M urea. 70ml of this mix was taken and polymerised by the addition of 22µl TEMED and 650µl 10% ammonium persulphate. This was used to pour a standard 0.4mm sequencing gel. The gel was run in 1x TBE buffer.

Gels were pre-run for 30 minutes at 1.5kV. Standard sharks tooth combs were used and 2.5µl of each sample containing 5000cpm/µl in 95% formamide, 20mM EDTA, 0.05% bromophenol blue and xylene cyanol FF was loaded per well. Gels were run for 2 hours at 2.8kV. Gels were fixed in 10% acetic acid: 10% methanol and dried onto Whatman 3MM paper. The dried gel was then exposed to βmax film (Amersham).

### Results

Control experiments were set up using the DNA primers and 18mer template. The DNA oligo primer sizes were 4mer, 5mer, 7mer, 9mer, 12mer, & 15mer respectively. Only the 9, 12, and 15mer gave efficient priming of the template as followed by the incorporation of [α³²P]dNTP on TLC plates. The 7mer did prime the template allowing extension but to a lesser degree than the larger DNA oligo's. This was true when both Klenow and exonuclease free Klenow enzymes were used respectively.

Further experiments used the PNA-DNA chimeric primers to prime the template, again priming of the template was followed by the incorporation of [α³²P]dNTP when analysed byTLC. Only the PNA-DNA 5mer failed to show any incorporation of the radiolabel. All the other four chimeras showed efficient incorporation of the radiolabel in the presence of either Klenow and dATPαS or exonuclease free Klenow.

The mobility of the labelled reaction products from the PNA-DNA chimera was significantly retarded and ran at a higher molecular weight compared to the corresponding DNA primer reaction products in polyacrylamide gel electrophoresis.

Competition extension reactions involving the equivalent chimera and DNA primers in the presence of the template resulted in the chimera being preferentially radiolabelled and no radiolabelled DNA product was produced.

Abbreviations
- DCM: dichloromethane
- DCC: dicyclohexylcarbodiimide
- DCU: dicyclohexylurea
- DECHA: diethylcyclohexylamine
- DMF: dimethylformamide
- EtOAc: ethyl acetate
- HBTU: hydroxybenzatriazolyltetramethyluroniumhexafluorophosphate
- MMTr: p-Anisoyldiphenylmethane
- HPLC: High Pressure Liquid Chromatography

## Claims

1. A method of performing a primer extension reaction by the use of
a) a target nucleic acid
b) a primer which is a chimeric nucleic acid/nucleic acid analogue molecule comprising a first portion, said first portion comprising a nonstandard backboned oligonucleotide and a second portion, said second portion comprising an acceptor end which is a chemical functionality capable of acting as acceptor for the formation of a phosphodiester bond, said chimeric molecule being capable of hybridising to part of the target
c) a supply of nucleotides
which method comprises mixing reagents a), b) and c) in the presence of a chain extension enzyme under conditions to allow the chimeric molecule to hybridise to the target and extension of the chimeric molecule at the acceptor end to occur, giving an extension product.

2. A method as claimed in claim 1, wherein at least one of reagents a), b) and c) is labelled.

3. A method of amplifying a target nucleic acid by an amplification reaction, wherein at least one primer is used which is a chimeric nucleic acid/nucleic acid analogue molecule comprising a first portion, said first portion comprising a nonstandard backboned oligonucleotide and a second portion, said second portion comprising an acceptor end which is a chemical functionality capable of acting as acceptor for the formation of a phosphodiester bond.

4. A method as claimed in claim 3, wherein the amplification reaction is the polymerase chain reaction.

5. A method of performing a chain termination reaction by the use of
a) a target nucleic acid
b) a primer which is a a chimeric nucleic acid/nucleic acid analogue molecule comprising a first portion, said first portion comprising a nonstandard backboned oligonucleotide and a second portion, said second portion comprising an acceptor end which is a chemical functionality capable of acting as acceptor for the formation of a phosphodiester bond, said chimeric molecule being capable of hybridising to part of the target
c) a supply of nucleotides
d) a chain termination agent
which method comprises mixing reagents a), b) c) and d) in the presence of a chain extension enzyme under conditions to allow the chimeric molecule to hybridise to the target and extension of the chimeric molecule at the acceptor end to occur, so as to produce terminated extension products, which terminated extension products are separated to allow part of the nucleotide sequence of the target nucleic acid to be determined.

6. A method as claimed in claim 5, wherein at least one of the reagents b), c) and d) is labelled.

7. A method of determining the nucleotide sequence of a target nucleic acid, which method comprises performing the method of claim 10 or claim 11 using a chain termination agent for each of the four different nucleotides such that the nucleotide sequence of the target may be determined.

8. A method as claimed in any one of claims 1 to 7, wherein the target nucleic acid is present in a double-stranded nucleic acid.

## Patentansprüche

1. Verfahren zur Durchführung einer Primer-Verlängerungsreaktion durch Verwendung von
a) einer Ziel-Nukleinsäure,
b) einem Primer, der ein chimäres Nukleinsäure/Nukleinsäure-Analoges-Molekül darstellt, umfassend einen ersten Teil, wobei der erste Teil ein kein Standardgerüst aufweisendes Oligonukleotid umfaßt, und einen zweiten Teil, wobei der zweite Teil ein Akzeptorende umfaßt, das eine chemische Funktionalität darstellt, die als Akzeptor zur Bildung einer Phosphodiesterbindung wirken kann, wobei das chimäre Molekül an einem Teil des Ziels hybridisieren kann;
c) einer Quelle für Nukleotide,
wobei das Verfahren Vermischen von Reagenzien a), b) und c) in Gegenwart eines Kettenverlängerungsenzyms unter Bedingungen, die es dem chimären Molekül ermöglichen, an dem Ziel zu hybridisieren und eine Verlängerung des chimären Moleküls an dem Akzeptorende unter Gewinnung eines Verlängerungsprodukts stattfinden zu lassen, umfaßt.

2. Verfahren nach Anspruch 1, wobei mindestens eines der Reagenzien a), b) und c) markiert ist.

3. Verfahren zur Amplifikation einer Ziel-Nukleinsäure durch eine Amplifikationsreaktion, wobei mindestens ein Primer verwendet wird, der ein chimäres Nukleinsäure/Nukleinsäure-Analoges-Molekül darstellt, umfassend einen ersten Teil, wobei der erste Teil ein kein Standardgerüst aufweisendes Oligonukleotid umfaßt, und einen zweiten Teil, wobei der zweite Teil ein Akzeptorende umfaßt, das eine chemische Funktionalität darstellt, die als Akzeptor zur Bildung einer Phosphodiesterbindung wirken kann.

4. Verfahren nach Anspruch 3, wobei die Amplifikationsreaktion die Polymerasenkettenreaktion ist.

5. Verfahren zur Durchführung einer Kettenbeendigungsreaktion durch die Verwendung von
a) einer Ziel-Nukleinsäure,
b) einem Primer, der ein chimäres Nukleinsäure/Nukleinsäure-Analoges-Molekül darstellt, umfassend einen ersten Teil, wobei der erste Teil ein kein Standardgerüst aufweisendes Oligonukleotid umfaßt, und einen zweiten Teil, wobei der zweite Teil ein Akzeptorende umfaßt, das eine chemische Funktionalität darstellt, die als Akzeptor zur Bildung einer Phosphodiesterbindung wirken kann, wobei das chimäre Molekül an einem Teil des Ziels hybridisieren kann;
c) einer Quelle für Nukleotide,
d) einem Kettenbeendigungsmittel
wobei das Verfahren Vermischen von Reagenzien a), b), c) und d) in Gegenwart eines Kettenverlängerungsenzyms unter Bedingungen, die es dem chimären Molekül ermöglichen, an dem Ziel zu hybridisieren und eine Verlängerung des chimären Moleküls an dem Akzeptorende stattfinden zu lassen, umfaßt, so daß beendete Verlängerungsprodukte erzeugt werden, wobei die beendeten Verlängerungsprodukte abgetrennt werden, damit ein Teil der Nukleotidsequenz der zu bestimmenden Ziel-Nukleinsäure abgetrennt werden kann.

6. Verfahren nach Anspruch 5, wobei mindestens eines der Reagenzien b), c) und d) markiert ist.

7. Verfahren zur Bestimmung der Nukleotidsequenz einer Ziel-Nukleinsäure, wobei das Verfahren Durchführung des Verfahrens nach Anspruch 10 oder Anspruch 11 unter Verwendung eines Kettenbeendigungsmittels für jedes der vier verschiedenen Nukleotide umfaßt, so daß die Nukleotidsequenz des Ziels bestimmt werden kann.

8. Verfahren nach einem der Ansprüche 1 bis 7, wobei die Ziel-Nukleinsäure in einer doppelsträngigen Nukleinsäure vorliegt.

## Revendications

1. Procédé pour réaliser une réaction d'allongement d'amorce en utilisant :
a) un acide nucléique cible ;
b) une amorce qui est une molécule chimérique analogue d'acide nucléique/acide nucléique comprenant une première portion, ladite première portion comprenant un oligonucléotide ayant un squelette non classique et une deuxième portion, ladite deuxième portion comprenant une extrémité accepteur qui est une fonctionnalité chimique capable d'agir en tant qu'accepteur pour la formation d'une liaison phosphodiester, ladite molécule chimérique étant capable de s'hybrider à une partie de la cible ;
c) un stock de nucléotides
procédé qui consiste à mélanger les réactifs a), b) et c) en présence d'une enzyme d'allongement de chaîne dans des conditions pour permettre à la molécule chimérique de s'hybrider à la cible et que l'allongement de la molécule chimérique ait lieu au niveau de l'extrémité accepteur, donnant un produit d'allongement.

2. Procédé selon la revendication 1, dans lequel au moins un des réactifs a), b) et c) est marqué.

3. Procédé d'amplification d'un acide nucléique cible par une réaction d'amplification, dans lequel au moins une amorce est utilisée qui est une molécule chimérique analogue d'acide nucléique/acide nucléique comprenant une première portion, ladite première portion comprenant un oligonucléotide ayant un squelette non classique et une deuxième portion, ladite deuxième portion comprenant une extrémité accepteur qui est une fonctionnalité chimique capable d'agir comme accepteur pour la formation d'une liaison phosphodiester.

4. Procédé selon la revendication 3, dans lequel la réaction d'amplification est l'amplification en chaîne par réaction.

5. Procédé de réalisation d'une réaction à terminaison de chaîne en utilisant :
a) un acide nucléique cible ;
b) une amorce qui est une molécule chimérique analogue d'acide nucléique/acide nucléique comprenant une première portion, ladite première portion comprenant un oligonucléotide ayant un squelette non classique et une deuxième portion, ladite deuxième portion comprenant une extrémité accepteur qui est une fonctionnalité chimique capable d'agir en tant qu'accepteur pour la formation d'une liaison phosphodiester, ladite molécule chimérique étant capable de s'hybrider à une partie de la cible ;
c) un stock de nucléotides ;
d) un agent de terminaison de chaîne,
procédé qui consiste à mélanger les réactifs a), b), c) et d) en présence d'une enzyme d'allongement de chaîne dans des conditions pour permettre à la molécule chimérique de s'hybrider à la cible et que l'allongement de la molécule chimérique ait lieu au niveau de l'extrémité accepteur, de façon à produire des produits d'allongement terminés, produits d'allongement terminés qui sont séparés pour permettre à une partie de la séquence de nucléotides de l'acide nucléique cible d'être déterminée.

6. Procédé selon la revendication 5, dans lequel au moins un des réactifs b), c) et d) est marqué.

7. Procédé pour déterminer la séquence de nucléotides d'un acide nucléique cible, procédé qui consiste à réaliser le procédé de la revendication 10 ou 11 en utilisant un agent de terminaison de chaîne pour chacun des quatre nucléotides différents de sorte que la séquence de nucléotides de la cible peut être déterminée.

8. Procédé selon l'une quelconque des revendications 1 à 7, dans lequel l'acide nucléique cible est présent dans un acide nucléique double-brin.
